# EUROPEAN PATENT APPLICATION

(11) **EP 4 699 617 A1**
(43) Date of publication of application: **25.02.2026**
(21) Application number: 24195926.1
(22) Date of filing: 22.08.2024
(51) Int. Cl.: A61K 39/108, A61P 1/00

(54) **ORAL VACCINE AGAINST MULTIPLE DIARRHEA-CAUSING PATHOGENS**

(71) Applicant: Scandinavian Biopharma Holding AB, 171 48 Solna (SE)
(72) Inventor: SJÖSTRAND, Björn, 168 50 Bromma (SE); CARLIN, Nils, 168 39 Bromma (SE)
(74) Representative: Novitas Patent AB

(57) **Abstract**

A vaccine comprising effective amounts of i) *E. coli* colonization factor antigens comprising CFA/I, CS3, CS5 and CS6 antigens; ii) a heat labile enterotoxin B subunit antigen being an *LCTBA* protein; and iii) an adjuvant being double mutant (R192G/ L211A) of *E. coli* heat-labile toxin (dmLT), for use in inducing protective immunity against diarrheal disease caused by *Salmonella spp.* and/or *Shigella spp.*

## Description

### TECHNICAL FIELD

The present invention relates to oral vaccines for use in the prevention of diarrheal disease caused by *Salmonella spp.* and/or *Shigella spp.*

### BACKGROUND TO THE INVENTION

The global diarrheal disease burden remains high, with approximately four billion cases estimated to occur annually in all age groups, with the highest incidence among infants and young children under five years of age. In this age group, enteric infections result in nearly 600,000 deaths each year, comprising approximately 9 percent of global child deaths and extracting an enormous physical and economic toll in low resource countries. Indeed, diarrhea is second only to pneumonia among the leading causes of death due to disease in children under five years old. Diarrhea can also be a triggering event for death from other causes, particularly pneumonia. Despite encouraging declines in overall diarrhea-associated mortality since year 2000, incidence rates remain high and have changed very little, even with incremental improvements in sanitation and water quality.

Travelers' diarrhea (TD) is the most common travel-related illness with per trip attack rate estimates of 10-40%. TD is a clinical syndrome that can result from a variety of intestinal pathogens. The predominant enteropathogens are bacteria that are considered to account for more than 80%-90% of cases. Overall, the most common pathogen identified is enterotoxigenic *Escherichia coli* (ETEC), followed by *Campylobacter jejuni, Shigella spp.,* and *Salmonella spp.* Enteroaggregative and other *E. coli* pathotypes also are commonly found in cases of TD. ETEC is especially prevalent in Latin America and African countries, but less so in Southeast Asia and is estimated to cause approximately 12 million episodes of travelers' diarrhea yearly. Infections with intestinal viruses including astrovirus, norovirus, and rotavirus, account for 5%-15% of TD illnesses. Infections with protozoal pathogens are slower to manifest symptoms and collectively account for around 10% of diagnoses in longer-term travelers. The main protozoal pathogen found in TD is Giardia. Entamoeba histolytica and Cryptosporidium are relatively uncommon causes of TD.

Among the primary infectious causes of diarrheal disease, enterotoxigenic *E. coli* (ETEC) is one of the most important pathogens for which there is currently no licensed vaccine. ETEC has been a long-standing World Health Organization (WHO) target for vaccine development. ETEC is also among the most frequent bacterial causes of diarrhea among travelers to Africa, Asia, and Latin America, including military personnel deployed to these areas.

The World Health Organization (WHO) describes *Salmonella* as one of the four most important causes of diarrhea worldwide. Every year, between 200 million to 1 billion cases of *Salmonella* infections are reported worldwide leading to 93 million cases of gastroenteritis and 155 000 deaths. In a smaller number of persons with *Salmonella* infections can develop to reactive arthritis pain with pain in the joints, irritation of the eyes, and painful urination. This disease with a duration between mounts or years is very difficult to treat. The severity of the disease depends on host factors and the subspecies and serovar of *Salmonella.* Among all the subspecies of *Salmonella* causing infection in humans *S. enterica subsp. enterica* (*I*) contributes approximately 99% of the infections. Gastroenteritis is caused by S. enterica serovars *Typhimurium (S. Typhimurium)* and *Enteritidis (S. Enteritidis)* and typhoid and paratyphoid fever, collectively named enteric fever, are contracted following infection with *S*. *enterica* serovars *Typhi (S. Typhi)* and *Paratyphi (S. Paratyphi),* respectively. Salmonella infections with invasive serovars are often life threatening, requiring effective antibiotic treatment. During recent years the emergence of multi-drug resistant (MDR) Salmonella serovars have been reported having a large impact on the efficacy of antibiotic treatment leading to an increase in mortality rates of *Salmonella* infections. Importantly, epidemiological studies indicate that MDR *Salmonella* serovars are more virulent than susceptible strains, resulting in an increased severity and more prolonged disease in patients infected by MDR strains. Preventive measures have been proposed to eliminate the spread of *Salmonella* infection and vaccine development is one important component of these preventive measures. Today approved vaccines against *Salmonella typhi* but not to other serovars is available.

Recent estimates attribute *Shigella* to cause approximately 125 million diarrhoeal episodes annually, leading to around 160000 deaths, with a third of these associated with young children. Shigellosis is generally a self-limiting infection but is also known to cause potential complications, most severely encephalopathy. Therefore, antibiotic treatment is recommended to prevent further complications, reduce diarrhoeal output, and limit post symptomatic faecal shedding. Unfortunately, resistance to antibiotic is reported to arise comparatively easy in *Shigella* and may be a consequence of an unrestricted barrier for horizontal gene transfer between *Shigella* and other Enterobacteriaceae. Novel treatments and vaccines are considered to be highly important to reduce the disease burden of this bacterial infection.

### Clinical development of ETVAX

ETVAX is a new multivalent, second-generation vaccine which is now under clinical development (WO2013037397A1). This improved vaccine contains 4 different inactivated *E*. *coli* strains expressing the most prevalent ETEC colonization factors (CFs), i.e. CFA/I, CS3, CS5 and CS6, in a total of approx. 8×10¹⁰ bacteria + 1 mg *LCTBA* and a mucosal adjuvant, i.e. double mutated LT (dmLT) (10 µg/dose) in a "full"/"normal" unit dose. Overall, ETEC vaccine administered with dmLT was safe and well tolerated when studied in several phase I and II clinical studies. No serious AEs were identified, and the recorded AEs were mainly mild. Immune responses analysed showed the tetravalent ETEC vaccine to be strongly immunogenic with intestinal (fecal) antibody responses and the intestine-derived antibody secreting cell (ALS) responses against the five primary antigens (LTB, CFA/I, CS3, CS5 and CS6) significantly higher in both frequency and magnitude in all three vaccine groups compared with the placebo group.

ETVAX has been studied in a randomized, placebo-controlled phase Ilb study to evaluate safety, immunogenicity, diagnostic methodology, and estimate vaccine efficacy of an oral enterotoxigenic *Escherichia coli* (ETEC) Vaccine (ETVAX) for prevention of clinically significant ETEC diarrhea in 729 healthy adult travelers visiting West Africa.

Furthermore, ETVAX has been studied in a phase IIB study in children 6-18 months in The Gambia. The objective of the study was to demonstrate if ETVAX^{®}, given with two doses 14 days apart and a booster dose at day 90, is safe and provides protection against moderate-to-severe diarrhoea caused by ETEC. The study was a randomized, placebo-controlled, double-blind, field trial of the vaccine in approximately 4936 children residing in an ETEC-endemic area in The Gambia.

### Object of the present invention

An object of the present invention is the provision of new methods for the reducing the risk of diarrheal disease caused by *Shigella spp.* and/or *Salmonella spp.* Another object is the provision of new uses for known vaccine compositions.

### DEFINITIONS

The term **antigen** refers to structures to which antibodies generated by the adaptive immune system of vertebrates specifically bind, or to which specific T-cell responses are elicited. Antigens are often proteins, peptides or polysaccharides (sometimes containing a lipid moiety). In the context of vaccines, an antigen is an ingredient in a vaccine against which a specific antibody and/or T-cell response is intended to be elicited in the host to which the vaccine is administered. Some vaccines contain several antigens. In the vaccine context, a vaccine antigen generally bears a structural resemblance to a pathogen antigen present in a pathogen against which the vaccine is intended to be used. A vaccine antigen may be a fragment or a modified version of the pathogen antigen, or a complete pathogen antigen presented in a non-pathogenic form e.g. as an inactivated/weakened pathogen or a recombinant protein produced in a non-pathogenic host.

The term **adjuvant** refers to a component added to a vaccine or other immunogenic composition that increases the effectiveness of the immune response to the antigen present in the vaccine or the other immunogenic composition.

The term **protective immunity** in the present context refers to immunization measures resulting in any degree of reduction in the likelihood of developing the condition for which the protective immunity is relevant, including a minor, substantial or major reduction in likelihood of developing the condition as well as total prevention. Preferably, the degree of likelihood reduction is at least a minor reduction.

**Colonization factors (CFs)** are major virulence factors of enterotoxigenic *Escherichia coli* (ETEC). In addition to colonization factors, ETEC virulence factors include heat-stable (ST) and heat-labile (LT) toxins. The CFs are either fimbrial, fibrial, or nonfimbrial in structure, and facilitate the adhesion of the bacteria to the intestinal cells and enable the bacteria to colonize the host. After colonization of the small intestine the bacteria secrete heat-labile toxin (LT), heat stable-toxin (ST) or both LT and ST which cause diarrhea. The morphology of the colonization factors is often determined by electron microscopy or predictions based on protein sequence.

The **CFA/I antigen, CS3 antigen, CS5 antigen** and **CS6 antigen** are prominent colonization factors present in ETEC isolates. A degree of biological sequence diversity exists in different strains and isolates and the sequence of an antigen useful in vaccines may differ somewhat from the reference sequence, as long as the antigen is capable of eliciting a clinically relevant immune response to the relevant colonization factor. The antigens have preferably at least 80%, or in increasing order of preference at least 85%, at least 95%, at least 98%, or complete sequence identity to their respective reference sequences cited below. In the context of designing ETEC vaccines, the antigens elicit the following relevant immune reactivity :
- A CFA/I antigen is capable of eliciting immune reactivity against CFA/I expressing ETEC strains.
- A CS3 antigen is capable of eliciting immune reactivity against CS3 expressing strains such as CS1 + CS3, CS2+ CS3 and CS3 only expressing ETEC strains.
- A CS5 antigen is capable of eliciting immune reactivity against CS5 expressing strains such as CS5 + CS6 expressing ETEC strains.
- A CS6 antigen is capable of eliciting immune reactivity against CS6 expressing strains such as CS4 + CS6, CS5 + CS6 and CS6 only expressing ETEC strains.

The **CFA/I (Colonization Factor Antigen I)** is a fimbrial structure, which allows it to attach to the intestinal lining. The fimbriae are composed of two main subunits. The CfaB (major repeating subunit) forms the bulk of the fimbriae and provides the repeating structural unit and the CfaE (single tip subunit) located at the tip of the fimbriae plays a role in initial attachment to host cells. See Jordi BJ, Willshaw GA, van der Zeijst BA, Gaastra W. The complete nucleotide sequence of region 1 of the CFA/I fimbrial operon of human enterotoxigenic Escherichia coli. DNA Seq. 1992;2(4):257-63 for an introduction to CFA/1. Reference sequences of cfaB and cfaE are published in Genebank accession number M55661.

The **CS3** (Coli Surface Antigen 3) is part of the fibrial family of colonization factors compising of two subunits cstH and cstG forming the CS3 fimbriae. See Jalajakumari MB, Thomas CJ, Halter R, Manning PA. Genes for biosynthesis and assembly of CS3 pili of CFA/II enterotoxigenic Escherichia coli: novel regulation of pilus production by bypassing an amber codon. Mol Microbiol. 1989;3(12):1685-95 for an introduction to CS3. A reference sequence of cstH and cstG are described in US11,077,200 B2 (cstG - SEQ ID NO: 87. cstH - SEQ ID NO: 85).

The **CS5** (Coli Surface Antigen 5) is a helical with two fimbria (csfA and csfD) arranged into a helix colonization factor expressed by certain ETEC strains. CS5 is one of the most common CFs worldwide, along with CS6. See Duthy TG, Staendner LH, Manning PA, Heuzenroeder MW. CS5 Pilus Biosynthesis Genes from Enterotoxigenic Escherichia coli O115:H40. J Bacterial [Internet]. 1999;181(18):5847-51 for an introduction to CS5. Reference sequences of csfA and csfD are published in Genebank accession number AJ224079.

The **CS6** (Coli Surface Antigen 6) is a nonfimbrial outer membrane protein colonization factor. CS6 is a polymer of two protein subunits CssA and CssB, CssB binds to sulfatide and recognizes the extracellular matrix protein fibronectin, and CssA shows general adhesiveness. See Wolf MK, de Haan LA, Cassels FJ, Willshaw GA, Warren R, Boedeker EC, et al. The CS6 colonization factor of human enterotoxigenic Escherichia coli contains two heterologous major subunits. FEMS Microbiol Lett. 1997;148(1):35-42 for an introduction to CS6. Reference sequences of cssA and cssB are published in Genebank accession number U04844.

The synonymous terms ***LCTBA*** and **LCTBA-protein** refer to a hybrid protein between the B-subunit of the *E. coli* heat-labile enterotoxin (LTB) and the B-subunit of the cholera toxin (CTB). Seven amino acids in the CTB molecule have been replaced by amino acids at corresponding positions of the LTB molecule. The details in relation to LCTBA, see Lebens M, Shahabi V, Backstrom M, et al. 1996. Synthesis of hybrid molecules between heat-labile enterotoxin and cholera toxin B subunits: potential for use in a broad spectrum vaccine. Infect Immun 64:2144-2150) and WO1996034893A1.

**dmLT** is a derivative of enterotoxigenic *Escherichia coli* heat labile toxin (LT) that has been genetically modified by replacing the arginine at amino acid position 192 with glycine and the leucine at amino acid position 211 with alanine. For an introduction to dmLT see , John D. Clements and Elizabeth B. Norton The Mucosal Vaccine Adjuvant LT(R192G/L211A) or dmLT mSphere. 2018; Jul-Aug; 3(4): e00215-18*,* WO1996006627A1, WO1999047167A1 and US 6,033, 673). The protein has been synonymously designated LT(R192G/L211A) in the literature and has been extensively evaluated in pre-clinical animal studies for its ability to adjuvant the immune responses for co-administered antigens. For characterization see Norton et al. (Norton EB, Lawson LB, Freytag LC, Clements J D. Characterization of a mutant Escherichia coli heat-labile toxin, LT(R192G/L211A), as a safe and effective oral adjuvant. Clin Vaccine Immunol. 2011 Apr; 18(4):546-51.).

The term ***sequence identity*** expressed in percentage is defined as the value determined by comparing two optimally aligned sequences over a comparison window, wherein a portion of the sequence in the comparison window may comprise additions or deletions (i.e., gaps) as compared to the reference sequence (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Unless indicated otherwise, the comparison window is the entire length of the sequence being referred to. In this context, optimal alignment is the alignment produced by the BLASTP algorithm as implemented online by the US National Center for Biotechnology Information (see The NCBI Handbook, 2nd edition [https://www.ncbi.nlm.nih.gov/books/NBK143764/]), with the following input parameters: Word length=3, Matrix=BLOSUM62, Gap cost=11, Gap extension cost=1.

### SUMMARY OF THE INVENTION

The inventors have unexpectedly discovered that the vaccine candidate being developed for the prevention of diarrheal disease caused by enterotoxigenic *Escherichia coli* (ETEC) also resulted in significant protective effect against diarrheal disease caused by *Shigella spp.* and *Salmonella spp* (see Example 3).

The present invention relates to the following items. The subject matter disclosed in the items below should be regarded disclosed in the same manner as if the subject matter were disclosed in patent claims.
1. An oral vaccine comprising effective amounts of:
   a. *E. coli* colonization factor antigens comprising CFA/I, CS3, CS5 and CS6 antigens;
   b. a heat labile enterotoxin B subunit antigen being an *LCTBA* protein; and
   c. an adjuvant being double mutant (R192G/ L211A) of *E. coli* heat-labile toxin (dmLT);
   for use in inducing protective immunity against diarrheal disease caused by *Shigella spp.* and/or *Salmonella spp.*
2. The vaccine for use according to item 1, wherein the disease is caused by *Shigella spp.*
3. The vaccine for use according to item 1, wherein the disease is caused by *Salmonella spp.*
4. The vaccine for use according to any of the preceding items, wherein the vaccine is further for use in inducing protective immunity against diarrheal disease caused by enterotoxigenic *E. coli* (ETEC).
5. The vaccine for use according to any of the preceding items, wherein the *E. coli* colonization factor antigens are present on inactivated whole bacterial cells, preferably *E. coli* cells.
6. The vaccine for use according to any of the preceding items, wherein the vaccine comprises inactivated whole bacterial cells in an amount of at least about 1.0 × 10¹⁰ cells per unit dose, preferably at least about 2.0 × 10¹⁰ cells per unit dose, more preferably at least about 4 × 10¹⁰ cells per unit dose, most preferably at least about 4.8 × 10¹⁰ cells per unit dose.
7. The vaccine for use according to any of the preceding items, wherein the vaccine comprises inactivated whole bacterial cells in an amount of less than 12 × 10¹⁰ cells per unit dose, preferably less than 11 × 10¹⁰ cells per unit dose, most preferably no more than 10 × 10¹⁰ cells per unit dose.
8. The vaccine for use according to any of the preceding items, wherein the vaccine comprises dmLT in an amount of at least about 1.0 µg per unit dose, preferably at least about 2 µg per unit dose, more preferably at least about 4 µg per unit dose, most preferably at least about 8 µg per unit dose.
9. The vaccine for use according to any of the preceding items, wherein the vaccine comprises *LCTBA* protein in an amount of at least about 0.1 mg per unit dose, preferably at least about 0.2 mg per unit dose, more preferably at least about 0.4 mg per unit dose, most preferably at least about 0.8 mg per unit dose.
10. The vaccine for use according to any of the preceding items, wherein the vaccine comprises CFA/I an amount of at least about 0.04 mg per unit dose, preferably at least about 0.08 mg per unit dose, more preferably at least about 0.15 mg per unit dose, most preferably at least about 0.3 mg per unit dose.
11. The vaccine for use according to any of the preceding items, wherein the vaccine comprises CS3 an amount of at least about 0.13 mg per unit dose, preferably at least about 0.25 mg per unit dose, more preferably at least about 0.5 mg per unit dose, most preferably at least about 1.0 mg per unit dose.
12. The vaccine for use according to any of the preceding items, wherein the vaccine comprises CS5 an amount of at least about 0.03 mg per unit dose, preferably at least about 0.05 mg per unit dose, more preferably at least about 0.1 mg per unit dose, most preferably at least about 0.2 mg per unit dose.
13. The vaccine for use according to any of the preceding items, wherein the vaccine comprises CS6 an amount of at least about 0.006 mg per unit dose, preferably at least about 0.013 mg per unit dose, more preferably at least about 0.025 mg per unit dose, most preferably at least about 0.05 mg per unit dose.
14. The vaccine for use according to any of the preceding items, wherein the vaccine comprises, per unit dose:
   a. inactivated whole bacterial cells comprising the following antigens:
      i. 0.3-1.6 mg CFA/I;
      ii. 1.0-5.2 mg CS3;
      iii. 0.2-1.1 mg CS5; and
      iv. 0.05-0.24 mg CS6;
   b. 0.8-1.2 mg of LCBTA protein; and
   c. 8-14 µg of dmLT adjuvant.
15. The vaccine for use according to item 14, wherein the unit dose comprises 4.0 × 10¹⁰ to 12 × 10¹⁰ whole cells.
16. The vaccine for use according to item 14 or 15, for use in immunizing human subjects of at least 6 years of age.
17. The vaccine for use according to any of items 1-13, wherein the vaccine comprises, per unit dose:
   a. inactivated whole bacterial cells comprising the following antigens:
      i. 0.15-0.8 mg CFA/I;
      ii. 0.5-2.6 mg CS3;
      iii. 0.1-0.55 mg CS5; and
      iv. 0.025-0.12 mg CS6;
   b. 0.4-0.6 mg of LCBTA protein; and
   c. 4-7 µg of dmLT adjuvant.
18. The vaccine for use according to item 17, wherein the unit dose comprises 2.0x 10¹⁰ to 6.0 × 10¹⁰ whole cells.
19. The vaccine for use according to item 17 or 18, for use in immunizing human subjects of 2-5 years of age.
20. The vaccine for use according to any of items 1-13, wherein the vaccine comprises, per unit dose:
   a. inactivated whole bacterial cells comprising the following antigens:
      i. 0.075-0.4 mg CFA/I;
      ii. 0.25-1.3 mg CS3;
      iii. 0.05-0.275 mg CS5; and
      iv. 0.0125-0.06 mg CS6;
   b. 0.2-0.3 mg of LCBTA protein; and
   c. 2-3.5 µg of dmLT adjuvant.
21. The vaccine for use according to item 20, wherein the unit dose comprises 1.0x 10¹⁰ to 3.0 × 10¹⁰ whole cells.
22. The vaccine for use according to item 20 or 21, for use in immunizing human subjects of 6-23 months of age.
23. The vaccine for use according to any of the preceding items, wherein the vaccine comprises inactivated *E. coli* cells comprising CS6 inactivated by phenol.
24. The vaccine for use according to any of the preceding items, wherein the vaccine is for administration in at least two doses separated in time by 7-30 days, preferably 14±7 days to a subject.
25. The vaccine for use according to any of the preceding items, wherein the vaccine is for use in oral administration to a subject in a buffer suitable for neutralizing gastric acid.
26. The vaccine for use according to any of the preceding items, wherein the vaccine is for use in a human subject.
27. The vaccine for use according to any of the preceding items, wherein the vaccine is for use in an adult human of at least 16 years of age.
28. The vaccine for use according to any of the preceding items, wherein the vaccine is for use in a human of 6-15 years of age.
29. The vaccine for use according to any of the preceding items, wherein the vaccine is for use in a human of 2-5 years of age at half of the unit dose.
30. The vaccine for use according to any of the preceding items, wherein the vaccine is for use in a human of 6-23 months of age at quarter of the unit dose.

### DETAILED DESCRIPTION

The present invention provides an oral vaccine comprising effective amounts of:
a. *E. coli* colonization factor antigens comprising CFA/I, CS3, CS5 and CS6 antigens;
b. a heat labile enterotoxin B subunit antigen being an *LCTBA* protein; and
c. an adjuvant being double mutant (R192G/ L211A) of *E. coli* heat-labile toxin (dmLT);
for use in inducing protective immunity against diarrheal disease caused by *Shigella spp.* and/or *Salmonella spp.*

The diarrheal disease may be caused by *Shigella spp.* Alternatively, the diarrheal disease is caused by *Salmonella spp.* The vaccine may further be for use in inducing protective immunity against diarrheal disease caused by enterotoxigenic *E. coli* (ETEC).

The *E. coli* colonization factor antigens may be present on inactivated whole bacterial cells, preferably *E. coli* cells.

The vaccine may comprise inactivated *E. coli* cells comprising CS6 inactivated by phenol. Suitable inactivation methods are disclosed in WO2013037718 A1. Other possible inactivation methods include formalin inactivation.

### Vaccine unit dose

The vaccine for the claimed use is normally provided in a unit dose. The amount of vaccine components in a unit dose may vary depending on the subject to be immunized. A unit dose intended for use in immunizing an adult human being at least 16 years of age as well as children and adolescents aged 6-15 years of age is considered a "full" unit dose or a "normal" unit dose. A unit dose for use in immunizing a human child of 2-5 years of age may contain half the amount of components compared to a full dose. A unit dose for use in immunizing a human infant of 6-23 months of age may contain a quarter of the amount of components compared to a full dose.

The vaccine may comprise inactivated whole bacterial cells in an amount of at least about 1.0 × 10¹⁰ cells per full unit dose, preferably at least about 2.0 × 10¹⁰ cells per full unit dose, more preferably at least about 4 × 10¹⁰ cells per full unit dose, most preferably at least about 4.8 × 10¹⁰ cells per full unit dose. The vaccine may comprise cells in an increasing order of preference, the following number of cells per full unit dose: about 0.8-14 × 10¹⁰, about 4.0-12 × 10¹⁰, about 4.8-10.0 × 10¹⁰ about 7.2-8.8 × 10¹⁰, about 8.0 × 10¹⁰. The vaccine may comprise inactivated whole bacterial cells in an amount of less than 12 × 10¹⁰ cells per full unit dose, preferably less than 11 × 10¹⁰ cells per full unit dose, most preferably no more than 10 × 10¹⁰ cells per full unit dose. For unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise dmLT in an amount of at least about 1.0 µg per full unit dose, preferably at least about 2 µg per full unit dose, more preferably at least about 4 µg per full unit dose, most preferably at least about 8 µg per full unit dose. The vaccine may comprise dmLT in an increasing order of preference, the following amount of dmLT per full unit dose: about 2.0-17.5 µg, about 4-15 µg, about 8-14 µg, about 9-11 µg, about 10 µg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise *LCTBA* protein in an amount of at least about 0.1 mg per full unit dose, preferably at least about 0.2 mg per full unit dose, more preferably at least about 0.4 mg per full unit dose, most preferably at least about 0.8 mg per full unit dose. The vaccine may comprise *LCTBA* protein, in an increasing order of preference, the following amount of *LCTBA* protein per full unit dose: about 0.20-1.5 mg, about 0.4-1.5 mg, about 0.8-1.2 mg, about 0.9-1.1 mg, about 1.0 mg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise colonization factor CFA/I an amount of at least about 0.04 mg per full unit dose, preferably at least about 0.08 mg per full unit dose, more preferably at least about 0.15 mg per full unit dose, most preferably at least about 0.3 mg per full unit dose. The vaccine may comprise CFA/I, in an increasing order of preference, the following amount of CFA/I per full unit dose: about 0.075-1.7 mg, about 0.15-1.6 mg, about 0.3-1.6 mg, about 0.9-1.1 mg, about 0.98 mg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise colonization factor CS3 an amount of at least about 0.13 mg per full unit dose, preferably at least about 0.25 mg per full unit dose, more preferably at least about 0.5 mg per full unit dose, most preferably at least about 1.0 mg per full unit dose. The vaccine may comprise CS3, an increasing order of preference, the following amount of CS3 per full unit dose: about 0.25-7.5 mg, about 0.5-6.0 mg, about 1.0-5.2 mg, about 2.8-3.4 mg, about 3.13 mg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise colonization factor CS5 an amount of at least about 0.03 mg per full unit dose, preferably at least about 0.05 mg per full unit dose, more preferably at least about 0.1 mg per full unit dose, most preferably at least about 0.2 mg per full unit dose. The vaccine may comprise CS5, in an increasing order of preference, the following amount of CS5 per full unit dose: about 0.05-2.0 mg, about 0.1-1.5 mg, about 0.2-1.1 mg, about 0.6-0.7 mg, about 0.63 mg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise colonization factor CS6 an amount of at least about 0.006 mg per full unit dose, preferably at least about 0.013 mg per full unit dose, more preferably at least about 0.025 mg per full unit dose, most preferably at least about 0.05 mg per full unit dose. The vaccine may comprise CS6, in an increasing order of preference, the following amount of CS6 per full unit dose: about 0.0125-0.5 mg, 0.025-0.5 mg, 0.05-0.24 mg, 0.13-0.16 mg, about 0.145 mg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

In an embodiment, the vaccine comprises, per unit dose:
a. inactivated whole bacterial cells comprising the following antigens:
   i. 0.3-1.6 mg CFA/I;
   ii. 1.0-5.2 mg CS3;
   iii. 0.2-1.1 mg CS5; and
   iv. 0.05-0.24 mg CS6;
b. 0.8-1.2 mg of LCBTA protein; and
c. 8-14 µg of dmLT adjuvant.

In this embodiment, the unit dose may comprise 4.0 × 10¹⁰ to 12 × 10¹⁰ whole cells.

The vaccine according to this embodiment may be for use in immunizing adult human subjects being at least 16 years of age as well as children and adolescents being 6-15 years of age.

In another embodiment, the vaccine comprises, per unit dose:
a. inactivated whole bacterial cells comprising the following antigens:
   i. 0.15-0.8 mg CFA/I;
   ii. 0.5-2.6 mg CS3;
   iii. 0.1-0.55 mg CS5; and
   iv. 0.025-0.12 mg CS6;
b. 0.4-0.6 mg of LCBTA protein; and
c. 4-7 µg of dmLT adjuvant.

In this embodiment, the unit dose may comprise 2.0x 10¹⁰ to 6.0 × 10¹⁰ whole cells.

The vaccine according to this embodiment may be for use in immunizing human subjects of 2-5 years of age.

In yet another embodiment, the vaccine comprises, per unit dose:
a. inactivated whole bacterial cells comprising the following antigens:
   i. 0.075-0.4 mg CFA/I;
   ii. 0.25-1.3 mg CS3;
   iii. 0.05-0.275 mg CS5; and
   iv. 0.0125-0.06 mg CS6;
b. 0.2-0.3 mg of LCBTA protein; and
c. 2-3.5 µg of dmLT adjuvant.

In this embodiment, the unit dose may comprise 1.0x 10¹⁰ to 3.0 × 10¹⁰ whole cells.

The vaccine according to this embodiment may be for use in immunizing human subjects of 6-23 months of age.

### Administration regimen and subjects

The vaccine may be formulated for oral administration to a subject, which is preferably a human.

The vaccine may be for oral administration to a subject in a buffer suitable for neutralizing gastric acid, such as a bicarbonate buffer solution, which may be effervescent.

For vaccines intended for adult human subjects being at least 16 years of age as well as children and adolescents being 6-15 years of age a "full" unit dose or a "normal" unit dose may be administered.

For vaccines intended for children, the unit dose as disclosed above may be reduced to half. For vaccines intended for infants, the unit dose may be reduced to ¼. For avoidance of any ambiguity, this entails that the amounts recited above for unit dose are adjusted accordingly, to arrive to a unit dose for children or unit dose for infants.

The vaccine may be for administration to an adult human being at least 16 years of age. For such subjects, a full unit dose may be administered per occasion. The vaccine may be for administration in at least two doses taken orally about 2-weeks interval (at least 1 week but not more than 6 weeks apart). The immunization should be completed at least 1 week prior to potential exposure to pathogens.

For children and adolescents aged 6-15, the primary vaccination series may involve two doses taken orally about 2-weeks interval (at least 1 week but not more than 6 weeks apart). The immunization should be completed at least 1 week prior to potential exposure to pathogens.

For children aged 2 to 5 years, the primary vaccination series may involve three doses of half the amount of the adults. The 3 doses are to be administered orally with about 2-weeks interval (at least 1 week but not more than 6 weeks apart) and at least 1 week prior to potential exposure to pathogens.

Human infants being 6 to 23 months, the primary vaccination series may involve three doses of 1/4 the amount of the full (adult) unit dose. For the infants, the first 2 doses are to be administered with about 2-weeks interval (at least 1 week but not more than 6 weeks apart) and the third dose 3-5 months (preferably about 3 months) after the first dose.

### General statements relating to the present disclosure

The term "comprising" is to be interpreted as including, but not being limited to. All references are hereby incorporated by reference. The arrangement of the present disclosure into sections with headings and subheadings is merely to improve legibility and is not to be interpreted limiting in any way, in particular, the division does not in any way preclude or limit combining features under different headings and subheadings with each other.

As used herein, the term "about" refers to a value or parameter herein that includes (and describes) embodiments that are directed to that value or parameter per se. For example, description referring to "about 20" includes description of "20." Numeric ranges are inclusive of the numbers defining the range. Generally speaking, the term "about" refers to the indicated value of the variable and to all values of the variable that are within the experimental error of the indicated value (e.g., within the 95% confidence interval for the mean) or within 10 percent of the indicated value, whichever is greater.

### EXAMPLES

The following examples are not to be regarded as limiting. For further information on the experimental details, the skilled reader is directed to a separate section titled Materials and Methods.

### Example 1: Design on clinical study

A clinical study has been performed with the aim to primarily study the safety, and immunogenicity of the ETVAX vaccine. The secondary aim was to evaluate the efficacy of ETVAX in healthy adult travelers visiting Benin-West Africa when it comes to prevention of ETEC induced Travelers' Diarrhea (TD). The safety of this new vaccine was evaluated by collecting data on vaccine attributable adverse events. For evaluating the immunological response to the vaccine, the serum antibody responses to heat-labile enterotoxin B subunit (LTB) were measured. The efficacy data was collected by following the incidence of TD cases occurring during or following the travel period in Benin.

This was a phase IIb, double-blind, randomized (ratio 1:1, placebo-controlled study in healthy adults (age 18-65 years) evaluating the safety, immunogenicity and efficacy of a two-dose regimen of ETVAX oral vaccine. The study included a pre-travel phase with four pre-travel visits at Aava Travel Clinic / University of Helsinki. The test product (ETVAX or placebo) was given 14±7 days apart (two-dose regimen) and 7-30 days prior to travelling to West Africa, Grand Popo, Benin for at least 12 days. Blood samples were collected for immunological analyses, and routine stool samples for microbial analyses. During the travel-period in Benin, subjects attended 3 regular visits and additional visits in case of TD. The first visit was held within 48 hours of arrival to give instructions for diarrhea reporting and stool collection procedures. During and after their stay in Benin (for 12 days in Benin, and 12 days after), subjects recorded their diarrheal episodes, related symptoms, and concomitant medication on health cards (HC). At each episode of TD specific stool samples (3rd and 4th defecation) were collected and recorded together with the symptoms in the daily health card. The sub-investigator was contacted with low threshold 24/7 for reporting symptoms and for requesting advice or a request for permission to take medication. For participants needing a personal evaluation, an additional visit was paid by the MD sub-investigator, or the patient visited the study clinic for assessment of symptoms. A TD episode was defined as a new episode if it was preceded by at least 48 diarrhea- and symptom-free hours without intake of diarrhea medication (loperamide or racecadotril). Diarrhea episodes occurring after use of antibiotics were not scored and if antibiotics had been taken before diarrheal stool sampling, the subject was excluded from efficacy analyses. The adverse event reporting period for this study began upon receiving the first dose of investigational medicinal product (IMP) and ended the day the subject travelled to Benin or at withdrawal of the subject. After returning to Finland from Benin, a 30-day follow-up period was held including after being back in Finland for 1-6 days and then approx. 30 days after return to Finland. Routine blood and stool samples were collected after being back in Finland for 1-6 days. In case of diarrhea episode occurring within 12 days after returning to Finland, additional stool samples were collected.

### In the study the following definitions were used for reporting the severity grade of the Travelers' Diarrhea (TD):

Passing ≥3 unformed/liquid stools within 24 h together with having one or more of the following gastrointestinal symptoms: fever, abdominal pain, cramping, nausea, vomiting, light-headedness (feeling weak), malaise and/or bloody stool with a severity grade at least 1.

The following grading scheme was used in reporting the severity grade of the above-mentioned gastrointestinal symptoms:

**Table X. Gastrointestinal symptom grading scheme**

| **Symptom** | **Grading** | | | |
|---|---|---|---|---|
| | 0 | 1 | 2 | 3 |
| Vomiting | not present | 1-2 episodes within a 24-hour period | 3-4 episodes within a 24-hour period | ≥5 episodes within a 24-hour period |
| Fever | not present | ≥37.5°C and ≤38.0°C | >38.0°C and ≤39°C | >39.0°C |
| Bloody stool | not present | minute traces of visible blood in stool or toilet paper | one stool with abundant blood (clots or liquid) | >1 stool with abundant blood (clots of liquid) |
| Other symptoms (abdominal pain, cramping, nausea, light-headedness (feeling weak), malaise) | not present | noticeable but not interfering with normal daily activity | interferes with normal activity | prevents normal daily activity |

For reporting the intensity of diarrhea, a similar grading scheme was used:
0 - No symptoms
1 - Symptom(s) which is mild and does not interfere with my planned activities for the day
2 - Symptom(s) which is severe enough to interfere with but not completely prevent my planned activities for the day (Example: I prefer to stay at home instead of going for an excursion or shopping tour, participating in planned sports activity etc.; however, I may consider to meet with other subjects, take a short walk, listen to a lecture, or similar)
3 - Symptom(s) which is so severe that it makes my planned activities for the day impossible (Example: Have to stay in bed or at home to be close to a toilet in case of diarrhea)

For an episode of diarrhea, the following applied:
1. The diarrhea episode started with the first reported unformed/liquid (i.e. diarrhea characterization grade of at least 3) stool of such a 24-hour period that contained at least 3 unformed/liquid stools with diarrhea intensity grade at least 1.
2. The diarrhea episode lasted until the beginning of the first 48-hour gap/period free of any unformed/liquid stools. The diarrhea episode ended with the last unformed/liquid stool before that gap.

In addition to the previous requirements, an episode of Travelers' Diarrhea (TD) satisfied the following requirement:
3. At least on one calendar day of the episode, the subject had reported at least one solicited TD-related symptom (fever, abdominal pain, cramping, nausea, vomiting, light-headedness (feeling weak), malaise or bloody stool) with severity grade at least 1.

### Example 2: Vaccine composition for clinical study

In the study, a tetravalent ETEC vaccine (ETVAX) with *LCTBA* supplemented with an adjuvant, double mutant heat labile toxin (dmLT), and an effervescent powder for oral solution (bicarbonate buffer) was evaluated. The ETEC vaccine (ETVAX) has been described previously (WO2013037397A1, WO2013037718A1) and contains formaldehyde or phenol inactivated recombinant *E. coli* strains (ETEX 21-24) overexpressing colonization factors CFA/I, CS3, CS5, and CS6 and a recombinant protein *LCTBA,* a chimera between *E. coli* heat-labile enterotoxin B subunit (LTB) and Vibrio cholerae cholera toxin B-subunit (CTB). An adjuvant, an inactivated double mutant LT(R192G/ L211A) of wild-type *E. coli* heat-labile toxin (dmLT) is administered with the vaccine. ETVAX is intended to protect against disease by inducing anti-colonization factor immunity that prevents adherence of ETEC bacteria to gut mucosa and anti-LT immunity that prevents binding of the heat-labile enterotoxin to the intestinal epithelium. The immune response elicited by the vaccine has been shown to be enhanced by the use of the dmLT adjuvant.

One full ETVAX dose contained up to approx. 8 × 10¹⁰ inactivated bacteria (*E. coli* ETEX21, ETEX22, ETEX23 and ETEX 24) and 1 mg of recombinant protein *LCTBA* and 10 µg of adjuvant dmLT. The product formulation of ETVAX used in this and earlier clinical trials is a liquid suspension (a so-called wet formulation) of inactivated bacteria (*E. coli* ETEX21, ETEX22, ETEX23 and ETEX 24) and *LCTBA* in one vaccine ampoule as described below, freeze-dried dmLT adjuvant in a second vial (10 mg/dose), and effervescent sodium bicarbonate buffer granules in a separate sachet to neutralize gastric acid upon ingestion.

The ETVAX vaccine ampoule represented a full dose and contained the following:

| | |
|---|---|
| *E. coli* ETEX 21 | 0.98 mg CFA/I |
| *E. coli* ETEX 22 | 3.13 mg CS3 |
| *E. coli* ETEX23 | 0.63 mg CS5 |
| *E. coli* ETEX24 | 0.145 mg CS6 |
| *LCTBA:* | 1 mg |
| Phosphate buffered saline: | q.s.ad 3.30 ml |

The vaccine or placebo was administered in two-dose regimen after the screening period with 14±7 days between the doses. The second dose was administered 7-30 days before departure to Benin.

### Preparation and administration of the investigational medicinal product

For the group A (placebo), the sodium bicarbonate buffer was dissolved in 150 ml of cold water by an unblinded study person before administration.

For the group B (Active), the dmLT ampoule (700 µg) was reconstituted with 700 µl sterile water and let stand for approx. 5 minutes in ambient temperature before dissolving by careful swirling. dmLT solution (250 µl) was mixed with 1000 µl of Phosphate Buffered Saline (PBS) solution (APL, Sweden; Art No 323469) in a 2 ml Eppendorf-tube (Eppendorf, Low protein binding Art No 022431102) and refrigerated (2-8 C°). The solution was used within 6 hours (stable up to 8 hours if kept refrigerated).

Thereafter, the unblinded study person dissolved the sodium bicarbonate buffer in a glass of cold water (150 ml) and poured the content of the ETVAX vaccine ampoule into the bicarbonate solution. A total of 50 µl of diluted dmLT solution (200 µg/ml) was added to the solution and the solution was stirred, before administration to the subject.

Both vaccine and placebo were given to the subject by a blinded nurse.

### Example 3: Vaccine efficacy against Salmonella and Shigella

When *Salmonella spp.* was used as an endpoint, regardless of ETEC infections or other co-infections, the incidence among vaccine and placebo recipients of moderate to severe *Salmonella* cases was 2.4% (8/333) and 5.8% (20/346), respectively.
- The protection afforded was 58% (p=0.03*)

For severe *Salmonella* cases the incidence was 0.9% (3/333) and 2.6% (9/346) in vaccine and placebo recipients, respectively.
- The protection afforded was 65% (p=0.14*)
- Fishers exact

When *Shigella spp.* was used as an endpoint, regardless of ETEC infections or other co-infections, the incidence among vaccine and placebo recipients of moderate to severe *Shigella* cases was 3.9% (13/333) and 6.1% (21/346), respectively.
- The protection afforded was 36% (p=0.22)

For severe *Shigella* cases the incidence was 2.1% (7/333) and 3.2% (11/346) in vaccine and placebo recipients, respectively.
- The protection afforded was 34% (p=0.48)

In conclusion, the ETVAX vaccine unexpectedly demonstrated prevention of *Salmonella spp* and *Shigella spp* positive diarrhea positive diarrhea with or without co-infections in healthy adult travelers.

### MATERIALS AND METHODS

### Definitions of the Analysis Population and Vaccine Preventable Outcomes (VPO)

Per Protocol Set (PPS) included all participants who have consented, are randomized, and have correctly received both of the assigned vaccine doses (ETVAX or placebo), arrived in Benin and completed study progress visit procedures (as documented by the Investigator) that include completed Health card(HC), and after return, at least completed stools passage information up to the end of study surveillance visit.

### Definition of Salmonella spp Vaccine Preventable Outcome (VPO):

Moderate or severe TD with stool sample containing *Salmonella* regardless of ETEC infections or other co-infections.

An episode of TD was considered severe, if at least once during the episode, the subject had reported at least 6 unformed/liquid stools (i.e. diarrhea characterization grade at least 3) during 24 hours, diarrhea prevented daily activities (i.e. diarrhea intensity grade 3) at least on one day, and the subject had reported at least once during the episode a gastrointestinal symptom that was at least mild (i.e. symptom severity grade at least 1).

An episode of TD was considered moderate, if at least once during the episode, the subject had reported at least 4 unformed/liquid stools (i.e. diarrhea characterization grade at least 3) during 24 hours, diarrhea interfered with daily activities (i.e. diarrhea intensity grade at least 2) at least on one day, the subject had reported at least once during the episode a gastrointestinal symptom that was at least mild (i.e. symptom severity grade at least 1), and the episode did not meet criteria for severe TD.

The criteria which qualified moderate or severe TD did not have to be satisfied simultaneously (i.e. on the same calendar day), just during the same TD episode.

Episodes of TDs were considered as vaccine preventable outcome if they further satisfied the criteria outlined below:
1. An episode that was moderate or severe TD.
2. In at least one stool sample taken during the diarrhea episode (but before taking any of the prohibited medications), the following condition were met:
   a. Cultures of Salmonella was positive and confirmed by Vitek MS (mass spectrometry microbial identification that uses matrix-assisted laser desorption/ionization -time-of-flight (MALDI-TOF) technology) from the pure cultures, except for the pathogens already present in at least one routine pre-travel stool sample.

### Definition of Shigella spp Vaccine Preventable Outcome (VPO):

Moderate or severe TD with stool sample containing *Shigella* regardless of ETEC infections or other co-infections.

An episode of TD was considered severe, if at least once during the episode, the subject had reported at least 6 unformed/liquid stools (i.e. diarrhea characterization grade at least 3) during 24 hours, diarrhea prevented daily activities (i.e. diarrhea intensity grade 3) at least on one day, and the subject had reported at least once during the episode a gastrointestinal symptom that was at least mild (i.e. symptom severity grade at least 1).

An episode of TD was considered moderate, if at least once during the episode, the subject had reported at least 4 unformed/liquid stools (i.e. diarrhea characterization grade at least 3) during 24 hours, diarrhea interfered with daily activities (i.e. diarrhea intensity grade at least 2) at least on one day, the subject had reported at least once during the episode a gastrointestinal symptom that was at least mild (i.e. symptom severity grade at least 1), and the episode did not meet criteria for severe TD.

The criteria which qualified moderate or severe TD did not have to be satisfied simultaneously (i.e. on the same calendar day), just during the same TD episode.

Episodes of TDs were considered as vaccine preventable outcome if they further satisfied the criteria outlined below:
1. An episode that was moderate or severe TD.
2. In at least one stool sample taken during the diarrhea episode (but before taking any of the prohibited medications), the following condition were met:
   a. Cultures of Shigella was positive and confirmed by Vitek MS (mass spectrometry microbial identification that uses matrix-assisted laser desorption/ionization -time-of-flight (MALDI-TOF) technology) from the pure cultures, except for the pathogens already present in at least one routine pre-travel stool sample.

### Diagnostic measurements

### Fecal culture and biochemical identification-based methods.

The results of culture based and biochemical identification-based methods according to Clinical and Laboratory Standards Institute (CLSI) and European Committee on Antimicrobial Susceptibility Testing (EUCAST) guidelines served as a reference.

The on-site laboratory in Benin VKRC was responsible for primary culture of the stool samples as soon as possible after receipt of specimen as well as preparing aliquots of stool specimen that was frozen (-70 °C) in eNAT tubes.

Primary cultivation and identification of *Salmonella, Shigella, Campylobacter* and *Vibrio cholerae* was made on site in Benin. Pure cultures were frozen (-70 °C) in cryopreservation tubes (Tammer-Tutkan Maljat, Finland or BioMerieux, France) for later analysis.

The presence of *Salmonella, Shigella, Campylobacter* and *Vibrio cholerae* were confirmed from the pure cultures by Vitek MS (mass spectrometry microbial identification that uses matrix-assisted laser desorption/ionization - time-of-flight (MALDI-TOF) technology).

To cover diarrheagenic *E. coli* species properly, the stool samples were cultured using a selective culture plate and twelve different colonies with the typical color of *E. coli* colonies are picked with single use culture loops into separate cryopreservation and after that six first ones of those loops are used to inoculate also an eNAT tube. The presence of EAEC, EPEC, ETEC, EHEC and Shigella/EIEC were confirmed by qPCR (Amplidiag Bacterial GE test) from the eNAT tube with six colonies isolated from the selective culture. If the eNAT tube was ETEC positive, the first six cryopreservation tubes (each with a different *E. coli* colony) were subjected to DNA extraction and the Amplidiag Bacterial GE test.

The ETEC-positive cryopreservation tubes are sent to GU for LT/ST/CF analyses. LT and/or ST production were analysed by GM1 Enzyme-Linked ImmunoSorbent Assay (ELISA) LT and ST tests at GU. In addition, expression of colonization factors CFA/I, CS3, CS5, CS6, and additional CFs were analyzed by monoclonal antibody-based dot blot tests at GU.

### Nucleic acid test for bacterial pathogens (Amplidiag qPCR)

The procedure started with DNA extraction from the aliquot of eNAT stored stool sample by using molecular extraction and PCR setup system Amplidiag Easy (Mobidiag) together with Amplidiag Extraction kit (Mobidiag). Amplidiag kits (Bacterial GE, C.difficile, Stool parasite or Viral GE) were used to obtain applicable PCR mix for each assay. The DNA concentration measurements were performed with real-time PCR detection system (Bio-Rad CFX96). If the eNAT tube was ETEC positive, the first six cryopreservation tubes (each with a different *E*. *coli* colony) were subjected to DNA extraction and the Amplidiag Bacterial GE test.

The extracted nucleic acids were used in various qualitative multiplex real-time PCR assays as follows:
- F-BactNAT: Bacterial enteric pathogens: Amplidiag Bacterial GE test (CE-IVD marked). The target panel included *Campylobacter, Salmonella, Shigella*/EIEC (enteroinvasive E. coli), Yersinia, EHEC (enterohemorrhagic E. coli), ETEC (enterotoxigenic E. coli), EPEC (enteropathogenic E. coli), and EAEC (enteroaggregative E. coli). Sensitivity of the test is 99.0% and specificity 99.9%.
- F-C.difficileNAT: Clostridium difficile: Amplidiag C. difficile+027 test (CE-IVD marked). The targets were C. difficile tcdB gene (toxin B gene) and novel 027 ribotype positive and 027 ribotype negative markers. Sensitivity of the test is 97.8% and specificity 100.0% for detection of the toxin B gene.
- F-ParasiteNAT: Enteric protozoa: Amplidiag Stool Parasite test (CE-IVD marked). The targets were *Cryptosporidium spp., Giardia lamblia, Entamoeba histolytica,* and *Dientamoeba fragilis.*
- F-ViraINAT: Gastrointestinal viruses: Amplidiag Viral GE test (CE-IVD marked) The targets were Noroviruses GI and GII, Rotavirus A, Sapovirus, Astrovirus, and Adenoviruses 40 and 41.

### Serum IgA and IgG antibody determinations

Vaccine-specific LTB and 078 IgA and IgG antibody levels were determined in serum samples collected before onset of immunization (day 1) and 5-6 days after second dose. The responses were analyzed with an electrochemiluminescence assay based on the Meso Scale Discovery (MSD) technology. (Akhtar M et al, 2019; Svennerholm AM et al 2022)

### Statistical methods & definition

Vaccine protective efficacy was derived from incidence ratios of diarrhea episodes with confidence intervals derived from 95% exact unconditional confidence limits of risk ratio, and p-values from Fisher's exact test.

## Claims

1. An oral vaccine comprising effective amounts of:
a. *E. coli* colonization factor antigens comprising CFA/I, CS3, CS5 and CS6 antigens;
b. a heat labile enterotoxin B subunit antigen being an *LCTBA* protein; and
c. an adjuvant being double mutant (R192G/ L211A) of *E. coli* heat-labile toxin (dmLT);
for use in inducing protective immunity against diarrheal disease caused by *Shigella spp.* and/or *Salmonella spp.*

2. The vaccine for use according to claim 1, wherein the disease is caused by *Shigella spp.*

3. The vaccine for use according to claim 1, wherein the disease is caused by *Salmonella spp.*

4. The vaccine for use according to any of the preceding claims, wherein the vaccine comprises:
a. dmLT in an amount of at least 1.0 µg per unit dose;
b. *LCTBA* protein in an amount of at least 0.1 mg per unit dose;
c. CFA/I an amount of at least 0.04 mg per unit dose;
d. CS3 an amount of at least 0.13 mg per unit dose;
e. CS5 an amount of at least 0.03 mg per unit dose; and
f. CS6 an amount of at least 0.006 mg per unit dose.

5. The vaccine for use according to any of the preceding claims, wherein the vaccine comprises inactivated whole bacterial cells in an amount of 1.0 × 10¹⁰ to 12 × 10¹⁰ cells per unit dose.

6. The vaccine for use according to any of the preceding claims, wherein the vaccine comprises, per unit dose:
a. inactivated whole bacterial cells comprising the following antigens:
i. 0.3-1.6 mg CFA/I;
ii. 1.0-5.2 mg CS3;
iii. 0.2-1.1 mg CS5; and
iv. 0.05-0.24 mg CS6;
b. 0.8-1.2 mg of LCBTA protein; and
c. 8-14 µg of dmLT adjuvant.

7. The vaccine for use according to claim 6, wherein the unit dose comprises 4.0 × 10¹⁰ to 12 × 10¹⁰ whole cells.

8. The vaccine for use according to claim 6 or 7, for use in immunizing human subjects of at least 6 years of age.

9. The vaccine for use according to any of claims 1-5, wherein the vaccine comprises, per unit dose:
a. inactivated whole bacterial cells comprising the following antigens:
i. 0.15-0.8 mg CFA/I;
ii. 0.5-2.6 mg CS3;
iii. 0.1-0.55 mg CS5; and
iv. 0.025-0.12 mg CS6;
b. 0.4-0.6 mg of LCBTA protein; and
c. 4-7 µg of dmLT adjuvant.

10. The vaccine for use according to claim 9, wherein the unit dose comprises 2.0x 10¹⁰ to 6.0 × 10¹⁰ whole cells.

11. The vaccine for use according to claim 9 or 10, for use in immunizing human subjects of 2-5 years of age.

12. The vaccine for use according to any of claims 1-5, wherein the vaccine comprises, per unit dose:
a. inactivated whole bacterial cells comprising the following antigens:
i. 0.075-0.4 mg CFA/I;
ii. 0.25-1.3 mg CS3;
iii. 0.05-0.275 mg CS5; and
iv. 0.0125-0.06 mg CS6;
b. 0.2-0.3 mg of LCBTA protein; and
c. 2-3.5 µg of dmLT adjuvant.

13. The vaccine for use according to claim 12, wherein the unit dose comprises 1.0x 10¹⁰ to 3.0 × 10¹⁰ whole cells.

14. The vaccine for use according to claim 12 or 13, for use in immunizing human subjects of 6-23 months of age.

15. The vaccine for use according to any of the preceding claims, wherein the vaccine is for administration in at least two doses separated in time by 7-30 days, preferably 14±7 days to a subject.
